# EUROPEAN PATENT APPLICATION

(11) **EP 4 197 565 A1**
(43) Date of publication of application: **21.06.2023**
(21) Application number: 21860615.0
(22) Date of filing: 12.08.2021
(51) Int. Cl.: A61L 9/015, A61L 9/03, A61L 9/20, A61L 2/00, A61L 2/20, C01B 13/10

(54) **CELING-MOUNTED OZONE-GENERATING DEVICE**

(30) Priority: 12.08.2020 ES 202031827 U
(71) Applicant: Oozbein Biohigiene, Sociedad Limitada, 41001 Sevilla (ES)
(72) Inventor: HERNANDEZ GARCIA, Luis Javier, 41001 Sevilla (ES)
(74) Representative: Garcia González, Sergio
(86) International application number: PCT/ES2021/070612
(87) International publication number: WO 2022/043594

(57) **Abstract**

The invention describes a ceiling-mounted ozone-generating device (1) which comprises: at least one ozone-generating element (2); a fan (3) arranged in a position adjacent to the ozone-generating element (2) to propel the ozone generated by the ozone- generating element (2) outwards; a processing means (4) that controls the operation of the ozone-generating element (2) and the fan (3); and a housing which comprises a cover (5a) and a base (5b) that houses the ozone-generating element (2), the fan (3), and the processing means (4), wherein the housing comprises an air inlet hole (6) and an ozone outlet hole (7). Additionally, the casing comprises means for fixing it to the ceiling of a room.

## Description

### Field of the Invention

The present disclosure relates to the field of interior disinfection, and more particularly to disinfection by means of ozone generation.

The object of the present invention is an ozone generating device designed for installation on the ceiling of a room.

### Background of the Invention

The health crisis generated by the appearance of the Covid-19 virus has caused a growing demand for devices capable of disinfecting rooms and surfaces, mainly indoors. Currently, there are various technologies used for this purpose, among which we can mention irradiation with ultraviolet light, spraying of disinfecting agents, or ozone generation.

Ozone is an enormously powerful and highly efficient disinfectant biocide against all types of microorganisms, such as viruses, bacteria and fungi, as well as for the elimination of odors and environmental hygiene in indoor spaces. The great instability of ozone molecules gives them a great oxidation capacity, so that they react quickly with the organic molecules of microorganisms. As a consequence, microorganisms are destroyed or degraded into smaller molecules, often less toxic or biodegradable. There are various mechanisms for the artificial generation of ozone (Os), among which are the methods of corona discharge with ceramic plates, electrolysis, radio-chemical or ultraviolet radiation.

Existing ozone generating devices have various drawbacks. First of all, in most cases these are portable devices designed to rest on a horizontal surface such as the floor, a table, a cabinet. This greatly reduces the safety in the application or use of ozone, since it forces ozone to saturate the environment because it, being heavier than air, tends to fall downwards once emitted. In addition, the devices known up to now hardly have any flexibility with regard to the emitted flow, nor do they allow it to be fully controlled, wirelessly by the user, nor do they work cyclically, that is, starting ozone action and later stop in production, to later repeat the programmed circle, this cyclical operation being a safety element as it is impossible to saturate an environment with ozone as a consequence of its own operation.

In addition, in the specific case of devices for disinfection of surfaces based on ultraviolet radiation, the presence of human beings is not allowed due to the wavelength of the emission, while with our device if possible due to continuous cycling system.

### Brief Description of the Invention

The present invention solves the previous problems thanks to a new ozone generation device that is specially designed for installation on the ceiling of a room. These characteristics constitute a great added value, because ozone weighs more than air, and at the same time a safety element, since with a lower production, better results are guaranteed due to the sole inertia of the own weight of the ozone. In addition, this device has additional advantages, since it allows for continuous control of the flow of ozone emitted, through its timing, programming its operation depending on the time or day, receiving power at several different voltages, communicate with the user wirelessly, etc. All these advantages will become clear from the following description.

The ozone generator device for the ceiling of the present invention generally comprises the following elements: at least one ozone generating element; a fan arranged in a position adjacent to the ozone generating element to drive the ozone generated by said element outwards; a processing means controls the operation of the ozone generating element and the fan; and a house that houses the ozone generating element, the fan, and the processing means, and that further comprises a lid, a base, an air inlet hole, and an ozone outlet hole.

The ozone generating element can be of any type as long as it is capable of generating a sufficient volume of ozone for the disinfection of a room. The ozone generating element can be of any type among all those described above, including without limitation: a corona discharge based generator with ceramic plates, an electrolysis based generator, a generator based on radio-chemical effect, a generator based on ultraviolet radiation, or others. In a particularly preferred embodiment of the invention, the ozone generating element is based comprising one or more ozone generating ultraviolet bulbs.

The characteristics described up to now are known from various ozone generating devices known today. However, the device of the present invention differs from those because, in addition to the previous elements, it includes means for fixing it to the ceiling of a room.

In principle, these means can take on different configurations, although in an articulately preferred embodiment of the invention they are screw holes arranged in the base of the house. Hooking clips for false ceilings arranged on the sides of the base of the house can also be used as fixing means. Moreover, in a very particularly preferred manner, the device of the invention comprises both types of fixing means, both screw holes and hook clips for false ceilings, thus giving the device great versatility.

The arrangement of fixing means to the ceiling of a room is particularly advantageous because ozone, being heavier than air, tends to fall downwards once generated. For this reason, the installation of the ozone generation device of the invention on the ceiling of a room turns out to be a significant improvement in terms of sanitization and cleaning efficiency.

In a preferred embodiment of the invention, the device further comprises voltage adaptation means configured to adapt various types of direct and alternating voltage. These are voltage adaptation means known in the art to adapt, for example, voltages of 220 VAC, 125 VAC, or 12 VDC to the nominal power supply voltage of the other elements of the device, such as the fan, the means of processing, or the ozone generating element itself. These voltage adaptation means can include a transformer or a power supply, as well as the necessary conversion elements to satisfy these needs.

The provision of voltage adaptation means in the device is advantageous because it provides great flexibility when connecting it, allowing its compatibility with the most common alternating voltages worldwide (220 VAC and 125 VAC) and with a direct voltage level (12 VCC) common in means of transport, such as ambulances, airplanes, buses, etc.

According to another preferred embodiment of the invention, the device further comprises a safety switch configured to stop the operation of the device when it detects the opening of the house lid. In principle, the safety switch can be configured in many different ways, as long as it makes it possible to detect the decoupling between the base and the housing lid. For example, in a particularly preferred embodiment of the invention, the lid comprises a protrusion which, when said lid is correctly attached to the base, it keeps the safety switch pressed. However, when the lid separates from the base, the projection stops depressing the safety switch, which causes the device to stop.

This configuration makes it possible to avoid accidents caused by the high supply voltages that any ozone generating element needs. Indeed, any type of ozone generating element requires a voltage that can reach several kilovolts. This can be dangerous when a person opens the case to perform any task. In this way, when opening the case, the safety switch automatically disconnects the power when the opening of the lid is detected. No need to unplug the device.

In principle, the operation of the device can be controlled in different ways as long as it allows acting on the flow of ozonated air depending on the needs of each application. However, in another preferred embodiment of the device, the processing means controls the operation of the ozone generating element and the fan according to a pulse width modulation scheme. The control scheme using pulse width modulation, o Pulse Width Modulation (PWM), is a well-known control strategy that is based on applying control pulses whose width is chosen based on the desired level of operation.

This configuration is particularly advantageous because it allows precise control of the ozonated air flow emitted at all times, thus allowing the level of operation to be adjusted to the needs of each specific application.

According to another preferred embodiment of the invention, the device further comprises a wireless communication module configured to communicate with a smart device to enable management of the device through an application installed on the smart device. For example, the wireless communication module may comprise a Bluetooth module, a WiFi module, or a combination of both. In this context, "smart device" refers to any device with processing and communication capabilities including, without limitation, a smartphone, tablet, smartwatch, computer, etc.

This configuration is advantageous because it facilitates the control of the device by the user. To do this, all you have to do is download a control application for the ozone generation device of the invention to your mobile phone and pair the phone with the device. The user can then control various operating parameters of the device, such as flow rate or on/off programming depending on the time or day of the week.

According to another preferred embodiment of the invention, the device incorporates a temperature sensor. This is an important safety element, since in the event of possible overheating, when a certain temperature is reached, for example 75°C, the ozone generating element turns off automatically and it does not work again until its temperature returns to normal, for example when it reaches 25°C. The user will always have knowledge of the state of the device through the means of communication.

According to another preferred embodiment of the invention the processing means is configured to operate the device continuously cyclically. This means that ozone will be produced at time intervals, pre-programmed in your software, to then produce a situation of stoppage or no ozone production, also pre-programmed. After this stop time, the process will be repeated, and this will be the case throughout the useful life of the device. In this way, it is guaranteed to avoid ambient saturation produced by an excess of ozone, since during the shutdown time, it decomposes and produces oxygen in its purest state.

In a further preferred embodiment of the invention, the device further comprises status LEDs configured to visually indicate the status of the device. Preferably, the LEDs comprise at least one green status LED indicating correct operation, a red status LED indicating a malfunction, and a blue status LED indicating wireless communication in progress. The LEDs, which are visible from the outside of the house, allow you to instantly identify the operating status of the device, and are also fireproof and self-extinguishing.

In another preferred embodiment of the invention, the device further comprises a removable filter arranged on an inner side of the air inlet. This makes it possible to prevent dust or foreign particles from entering the house, thus extending the useful life of the electronic components of the device.

### Brief Description of the Figures

Figure 1 shows a perspective view of the ozone generating device of the present invention.
Figure 2 shows another perspective view of the ozone generating device of the present invention.
Figure 3 shows yet another perspective view of the ozone generating device of the present invention.
Figure 4 shows a perspective view of the ozone generating device of the present invention with the house lid removed.
Figure 5 shows a schematic diagram of some of the elements that make up the device of the invention.

### Detailed Description of the invention

### Preferred Embodiment of the Invention

An example of ozone generating device (1) for ceilings according to the invention is described below with reference to the attached figures.

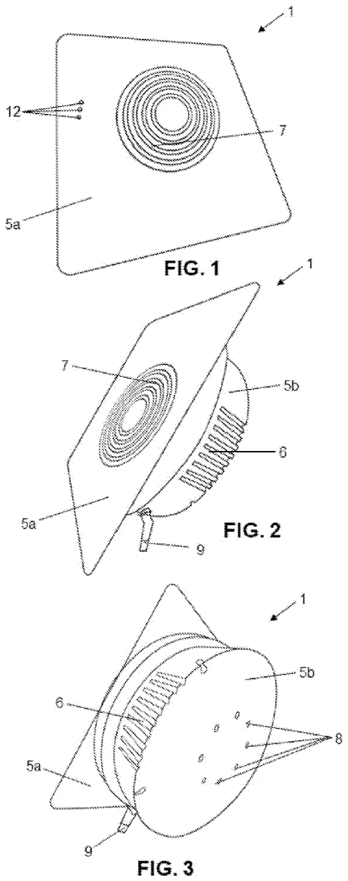

The figures 1, 2 and 3 show the external appearance of the device (1) of the invention. As can be seen, the device (1) comprises a house that is made up of two parts: a lid (5a) and a base (5b). The base (5b) has an essentially cylindrical shape formed by a cylindrical side wall closed on one side and open on the opposite side. The lid (5a) is in the shape of a flat plate to close the open side of the cylindrical base (5b). In this example, the lid (5a) has a square shape with dimensions substantially greater than the diameter of the base (5b), although other sizes and shapes are possible depending on the desired aesthetic appearance of the device (1).

On the closed side of the base (5b), formed by a circular bottom plate, there are holes (8) for screws designed for coupling the base (5b) to the ceiling of a room. In this example, there are eight holes (8) arranged according to a radial configuration around the axis of the cylindrical base (5b).

The base (5b) also has hooking clips (9) that protrude out of the cylindrical side wall. These clips (9) are configured to adopt two positions: a first position where they are parallel to the cylindrical side wall of the base (5b), and a second position where they are essentially perpendicular to the cylindrical wall of the base (5b). Thanks to this, it is possible to insert the base (5b) of the device (1), with the clips (9) in the first position, through a hole made for this purpose in the false ceiling of a room to then pass the clips (9) to the second position. The device (1) is thus fixed to the false ceiling quickly and safely.

The base (5b) of the house also has an air inlet hole (6) formed by a grill. After the air inlet hole (6), on an inner side thereof, there is a support designed to support a removable filter (13) so that it completely lids the hole (6). In this way, all the air that enters the interior of the house is filtered, eliminating dust and other foreign elements that could damage the electronics of the device (1). The figures also show an ozone outlet hole (7) arranged in a central portion of the lid (5a) of the house. This outlet hole (7) also takes the form of a grid, although in this case it is a circular grid with a gradual angle designed to have an expansion effect. Thus, the device (1) of the invention absorbs air through the inlet hole (6) and expels ozone, or more specifically ozonated air, through the outlet hole (7).

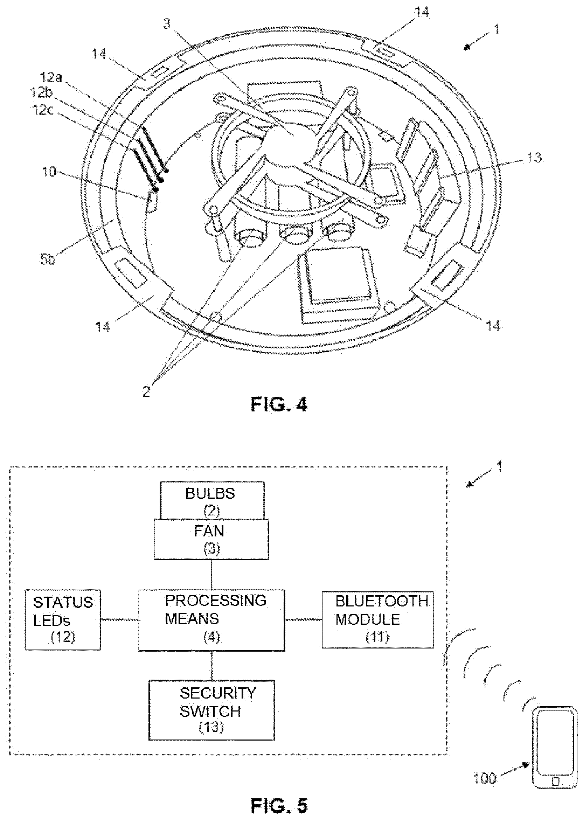

Figure 4 shows the device (1) of the invention with the lid (5a) of the house removed, so that the components housed inside the base (5b) can be seen. In this specific configuration, bulbs (2) arranged on a plate fixed to the bottom of the base (5b) of the house are used as an ozone generating element. These are ozone-emitting bulbs (2) that cause the decomposition of oxygen and the appearance of ozone according to a known procedure. Above the ozone generating bulbs (2) is the fan (3), in this case, an axial fan that drives the ozone generated in an axial direction to make it exit through the outlet hole (7) arranged in the lid (5a) of the house. As previously described, the hole grid (7) has an expansive effect that ensures the expansion of the ozone emitted throughout the room in which the lamp (1) is installed.

Figure 4 also shows the filter (13) of the air that enters the house through the inlet hole (6), as well as three LEDs (12a, 12b, 12c) that indicate the status of the device (1) at all times. Specifically, the green LED (12a) indicates correct operation, the red LED (12b) indicates a malfunction, and the blue LED (12c) indicates wireless communication in progress. In addition, a safety switch (10) can be seen arranged next to the side wall of the base (5b) of the house. A projection on the lid (5a) of the house (not shown in the figures) is positioned so that, when the lid (5a) is fixed to the base (5b) by means of the coupling elements (14), it keeps the switch (10) depressed. 5aOn the contrary, when the lid (5a) is disassembled to open the device (1), by separating said lid (5a) from the base (5b), the protrusion stops pressing the safety switch (10). This immediately causes the device (1) to stop working and the red alarm LED (12b) to light up. In this way, accidents due to inadvertent electrical contact with high-voltage parts are avoided.

For reasons of simplicity, in figure 4 various electronic elements defined previously in this document have not been represented, such as current matching means, processors, and in general electronic elements whose representation does not provide greater clarity to this description.

Finally, figure 5 shows a simplified schematic diagram of the invention where some of its main elements can be seen. As can be seen, the ozone-generating bulbs (2) and the fan (3) are connected to a processing means (4) that manages their operation. The processing means (4) can in principle be of any type, such as a microprocessor, a microcontroller, a DSP, etc. an FPGA, an ASIC, or others. The processing means (4) is also connected to the status LEDs (12) and the safety switch (10) shown in figure 4. Figure 5 also shows connected to the processing means (4) a Bluetooth module (11) responsible for wireless communication between the device (1) of the invention and an external device (100) such as a smartphone. Thanks to this, the user can act on the device (1) to, for example, turn it on and off, schedule its operation based on the day, hour, month, or any other time interval, and even control the flow of ozone emitted.

## Claims

1. Device (1) ozone generator for ceiling, comprising:
- at least one element (2) ozone generator;
- a fan (3) arranged in a position adjacent to the ozone-generating element (2) to drive the ozone generated by said element (2) outwards;
- a processing means (4) that controls the operation of the ozone generating element (2) and the fan (3);
- a house, comprising a cover (5a) and a base (5b), which houses the ozone-generating element (2), the fan (3) and the processing means (4), where the house comprises an air inlet hole (6) and an ozone outlet hole (7), **characterized in that** the house also comprises means for fixing it to the ceiling of a room.

2. Ceiling ozone generator device (1) according to claim 1, wherein the fixing means are screw holes (8) arranged in the base (5b) of the house (5b).

3. Ozone generator device (1) for the ceiling according to any of the preceding claims, wherein the fixing means are hook clips (9) for the false ceiling arranged on one side of the base (5b) of the house.

4. Ceiling ozone generator device (1) according to any of the preceding claims, further comprising voltage adaptation means configured to adapt various types of direct and alternating voltage.

5. Ozone generator device (1) for ceilings according to claim 4, wherein the voltages comprise: 220 VAC, 125 VAC, and 12 VCC.

6. Ceiling ozone generator device (1) according to any of the preceding claims, which also comprises a safety switch (10) configured to stop the operation of the device (1) when it detects the opening of the cover (5a) of the house.

7. Ceiling ozone generator device (1) according to claim 6, where the cover (5a) comprises a projection that, when said cover (5a) is correctly coupled to the base (5b), keeps the safety switch (10) depressed while, when the cover (5a) separates from the base (5b), the projection stops pressing the safety switch (10), which causes the device (1) to stop.

8. Ceiling ozone generator device (1) according to any of the preceding claims, wherein the processing means (4) controls the operation of the ozone generator element (2) and the fan (3) according to a pulse width modulation scheme.

9. Ceiling ozone generator device (1) according to any of the preceding claims, which also comprises a wireless communication module (11) configured to communicate with an intelligent device (100) to allow management of the device (1) through an application installed on said smart device (100).

10. Ozone generator device (1) for ceiling according to claim 9, where the wireless communication module (11) comprises a Bluetooth module, a WiFi module, or a combination of both.

11. Device (1) according to any of the preceding claims, which also comprises status LEDs (12a, 12b, 12c) configured to visually indicate the status of the device (1).

12. Device (1) according to claim 11, comprising a green status LED (12a) indicating correct operation, a red status LED (12b) indicating a malfunction, and a blue status LED (12c) indicating wireless communication in progress.

13. Device (1) according to any of the preceding claims, comprising a removable filter (13) arranged on an inner side of the air inlet hole (6).

14. Device (1) according to any of the preceding claims, which also comprises a temperature sensor.

15. Device (1) according to any of the preceding claims, wherein the ozone generating element (2) comprises one or more ozone generating ultraviolet bulbs.
